# EUROPEAN PATENT APPLICATION

(11) **EP 2 532 639 A1**
(43) Date of publication of application: **12.12.2012**
(21) Application number: 11169339.6
(22) Date of filing: 09.06.2011
(51) Int. Cl.: C07C 13/45, B01J 20/32, C08J 3/28, G01N 33/50

(54) **Method for preparing a reactive coating**

(71) Applicant: ModiQuest B.V., 6525 AJ Nijmegen (NL)
(72) Inventor: Van Hest, Jan Cornelis Maria, 6533 AC Nijmegen (NL); Canalle, Luiz Alberto, 5851 DK Afferden (NL); Salden, Martinus Hubertus Leonardus, 6536 CL Nijmegen (NL); Chirivi, Renato Gerardus Silvano, 4908 CR Oosterhout (NL); Raats, Jozef Maria Hendrik, 6525 XV Nijmegen (NL)
(74) Representative: Habets, Winand

(57) **Abstract**

This invention is in the field of immuno chemistry and organic chemistry and provides means and methods for immobilizing a molecule, more in particular an antigen to a solid support. This allows for the economic and easy provision of immuno-assays, such as enzyme-linked immunosorbent assays. More in particular, the invention provides a method for immobilizing a bicycloalkyne to a solid support wherein a UV-reactive monomer is attached to the bicycloalkyne to obtain a UV-reactive clickable monomer, where after the UV-reactive clickable monomer is covalently linked to the solid support by means of UV irradiation in the presence of a photoinitiator.

## Description

### Field of the invention

This invention is in the field of immuno chemistry and organic chemistry and provides means and methods for immobilizing a molecule, more in particular an antigen to a solid support. This allows for the economic and easy provision of immuno-assays, such as enzyme-linked immunosorbent assays.

### Background of the invention

The enzyme-linked immunosorbent assay (ELISA) [2] is a well-established biochemical technique that is used on a large scale as a diagnostic tool within medicine, like in pregnancy tests, and for quality control in various industries, for instance while monitoring food allergens. ELISA is based on the specific binding between binding partners such as an an antigen and its appropriate antibody, which can be quantified with an enzyme label on either binding partner. The principle of specific recognition of a particular region on the antigen called the epitope makes ELISA tests a valuable medical tool for the diagnosis of diseases such as infectious diseases or auto-immune diseases.

The ELISA procedure is particularly suited to detect one binding partner in solution when the other binding partner is immobilized on a solid support. A manifold of such supports are known and readily available in the art, polystyrene and nitrocellulose are commonly used. Immobilization to a solid support may be achieved by one of several methods applied in traditional ELISA, such as passive absorption, [6] chemical conjugation to reactive groups such as maleic anhydride [7] and non-covalent interaction such as for example between streptavidin or avidin and biotin [1]. However, these approaches suffer from serious disadvantages, such as that they are either very expensive or give little to no control over the attachment site of the peptide, which can ultimately reduce the sensitivity of the ELISA.

It is therefore an object of the present invention to provide cheap, reliable and convenient ways to ameliorate or solve the problems encountered with prior art immobilization procedures.

### Summary of the invention

It has now surprisingly been found that bicycloalkynes, more in particular bicyclononynes such as bicyclo[6.1.0]non-4-yne (BCN) can withstand UV irradiation and may therefore be advantageously used in a method of immobilizing an antigen, for instance in a copper-free clickable ELISA.

The invention therefore relates to a method for immobilizing a bicycloalkyne to a solid support wherein a UV-reactive monomer is attached to the bicycloalkyne to obtain a UV-reactive clickable monomer, where after the UV-reactive clickable monomer is covalently linked to a solid support by means of UV irradiation in the presence of a photoinitiator.

### Detailed description of the invention

Bicycloalkynes per se are known in the art. It has been described that such molecules may advantageously be used in what has been called click-chemistry. The chemoselective, robust and versatile 'click' reactions have already been successfully used in the construction and functionalization of polymeric coatings [14-21]. So far, these coatings were based on non-specific adsorption to a solid support.

Surprisingly, we have now found that bicycloalkynes can withstand UV irradiation to a level sufficient to covalently couple a bicycloalkyne-functionalised monomer to a solid support.

In one aspect, the invention therefore relates to a method for immobilizing a bicycloalkyne to a solid support wherein a UV-reactive monomer is attached to the bicycloalkyne to obtain a UV-reactive clickable monomer, where after the UV-reactive clickable monomer is covalently linked to a solid support by means of UV irradiation in the presence of a photoinitiator.

In a preferred method according to the invention, any molecule may subsequently be attached through a click-reaction to the bicycloalkyne immobilized on the solid support. In a preferred embodiment, the molecule carries complementary reactive groups with respect to the cycloalkynes, such as 1,3 dipolar compounds, such as azides, nitrile oxides and nitrones. In a method according to the invention, the molecule becomes covalently attached to the solid support through a covalent bond formed via a [3+2] cycloaddition reaction.

The solid support may be any support suitable for the purpose, although a flat substrate is preferred for ELISA purposes. The support may consist of inert material such as glass, paper, metal or polymers. If a polymer is to be used as a solid support, polystyrene or polymethylmetacrylate (PMMA) is preferred.

The term UV-reactive monomer is used to indicate any monomer capable of polymerizing under photochemical conditions. For certain applications, it may be advantageous to use fast-polymerising monomers which are known in the art. Suitable examples of such monomers are acrylates, methacrylates and acrylamides.

The conditions of UV irradiation may vary with the intended purpose. A skilled person is well aware of the meets and bounds of UV irradiation and will be able to select suitable criteria for use in the method as described herein. In the examples described herein, a UVA Spot 400T bulb was used during 15 minutes at 20 cm from the surface. This resulted in less than 5% decomposition of the strained alkyne.

The method according to the invention may be performed with any bicycloalkyne. In a preferred embodiment bicyclononynes are used. In a particularly preferred embodiment, the commercially available bicyclo[6.1.0]non-4-yne (BCN) may be used.

In that way, clickable coatings may be obtained wherein the bicycloalkyne is covalently attached to a solid support. Such supports may then advantageously be used to immobilize any other molecule that carries a suitable reactive group.

Because of the above mentioned disadvantages, we set out to develop an ELISA employing a new microtiter plate coating strategy based on the recently developed copper-free strain-promoted azide-alkyne 1,3-dipolar cycloaddition (SPAAC) [9-13].

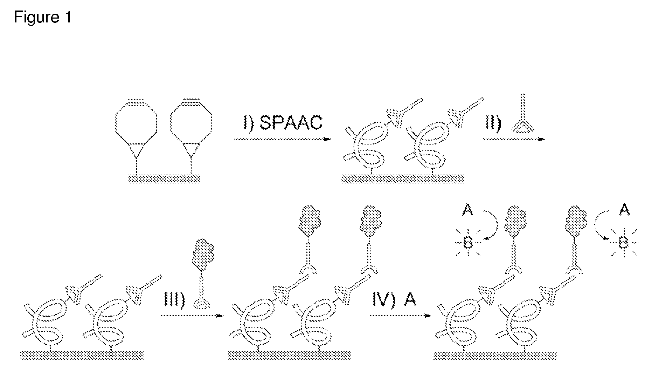

It is disclosed herein that SPAAC is a cost-effective and selective immobilization method in the production of a heterogeneous sandwich ELISA [22] that can detect disease specific proteins. The principle of this ELISA procedure is depicted in Figure 1.

Immunoassays, such as the ELISA as provided and exemplified herein are capable of detecting anti-citrulline antibodies that are characteristic for Rheumatoid Arthritis, a common human auto-immune disease, which is characterized by chronic inflammation of the joints. The inflammatory process results in the post-translational modification of a range of proteins. The immune system no longer recognizes these modifications as "self", and starts producing antibodies against the modified proteins. Although the exact pathogenic nature of the different antibodies is still under debate,[3] several antibodies have been identified that are exclusively associated with RA and can therefore serve as specific biomarkers. One of the most promising groups of antibodies are those against citrullinated proteins [4].

During a runaway inflammation process, dead cells release the enzyme peptidylarginine deiminase, which subsequently transforms arginine residues present in extracellular peptides into the neutral residue citrulline. As a consequence of continuous deimination of arginines, this process ultimately leads to the formation of autoimmune anti-citrulline antibodies (ACA's) [5] Because they are already present during the early stages of RA, the autoimmune human ACA's have a high predictive value for RA and can be used during early diagnosis and prognosis up to 10 years before the manifestation any clinical signs of the disease.

Bicycloalkynes may be attached to hydrophilic monomers in any conventional way known in the art. As an example, we provide herein the synthesis of a particular bicycloalkyne known as bicyclo[6.1.0]non-4-yne (BCN) attached to methacrylate. This BCN-decorated methacrylate was used in the ELISA based on SPAAC.

For that purpose, BCN was synthesized as a mixture of diastereomers (exo-1 and endo-1) according to literature procedure [13]. Because both stereoisomers exhibit similar reactivity toward azides [13] they were not separated and used as a mixture for the rest of the synthesis. As is shown in scheme 1, the hydroxyl group of 1 was subsequently reacted with an excess of methacryloyl chloride under basic conditions using 4-dimethylaminopyridine (DMAP) as a catalyst for the esterification reaction. In this way, BCN methacrylate 2 was obtained. 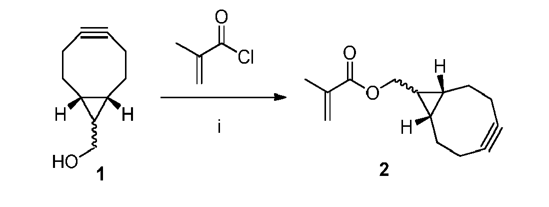 **Scheme 1.** Synthesis of monomers **2.** *Reagents and conditions:* i) Et₃N, DMAP, CH₂Cl₂, 0 ºC to r.t., 16 h (70%).

¹H-NMR analysis of a solution of BCN methacrylate 2 and Irgacure 1000 after UV exposure indicated that the strained alkyne system 2 was fully resistant towards the radicals generated during polymerisation.

For the ELISA, a monomer mixture was made containing 10% v/v of the BCN- methacrylate together with poly(ethylene glycol) dimethacrylate, ethylene glycol dimethacrylate and 2-hydroxyethyl methacrylate.

Poly(ethylene glycol) dimethacrylate may be added since it was found to advantageously serve as a cross-linker and provides a hydrophilic, water-swellable polymer network.

Ethylene glycol dimethacrylate may be added as a non-essential and additional cross-linker. This resulted in an advantageously better adhesion to the microtiter plate, presumably by interacting with the poly(styrene) substrate.

As another additional monomer component, 2-hydroxyethyl methacrylate may be used to lower the viscosity of the monomer mixture, making it easier to handle.

The final coating formulation was prepared by diluting the monomer mixture with methanol to a monomer content of 5% (v/v). The ratio of the different additional hydrophilic monomers may be varied in a simple optimization process. The stability, adhesion and transparency of the final coating may be influenced and fine-tuned in this way.

The final coating formulation may be filled into the wells of a microtiter plate, such as a standard 96 wells polystyrene microtiterplate. In this way a liquid film is formed on the walls of the microtiter plate wells.

The liquid film was polymerized using a strong UV source that was positioned 20 centimeters above the sample for 60 seconds. After 30 minutes the UV exposure was repeated in order to improve the clarity and stability of the coating.

Reactive peptide 5 with sequence LSEGGGVCitGPRVVECitHQSQCK (SEQ ID NO: 1) and control peptide 6 with sequence LSEGGGVRGPRVVERHQSQCK (SEQ ID NO: 2) were obtained as described in the examples section. Both peptides were provided with an azide function on their C-terminus. Both peptides were immobilized by filling the peptide solutions in DMSO/H₂O (1/2) into the microtiter wells thereby contacting the peptide solutions with the immobilized BCN-methacylate and allowing them to react for 2 hours at room temperature..

ELISA procedures were performed after washing the plates with PBS, blocking the microtiter plate using bovine serum albumin (BSA) followed by incubation with a solution of human anti-citrulline antibody MQR2.101 for 1 hour. This antibody is commercially available from ModiQuest BV, Nijmegen, The Netherlands. In the detection step, monoclonal rabbit anti-human antibody conjugated to the horseradish peroxidase enzyme (HRP) was added to the microtiter plates and incubated for 1 hour. The microtiter plates were finally developed with a 3,3',5,5'-tetramethylbenzidine (TMB) solution for 5 minutes in the dark. The absorbance at 450 nm is a direct measure of the activity of HRP bound to the microtiter plates, which in turn indicates how much anti-citrulline antibody was initially bound to the antigenic peptide immobilized on the walls of the polystyrene microtiter plate.

We observed that the peptides 5 and 6 were successfully immobilized on the microtiter plate. Peptide 5 showed a strong reactivity with the anti-citrulline antibodies whereas peptide 6 showed no significant reaction.

These results illustrate that this BCN-based immobilization technology is suitable for diagnostic purposes, in particular for sandwich ELISA's.

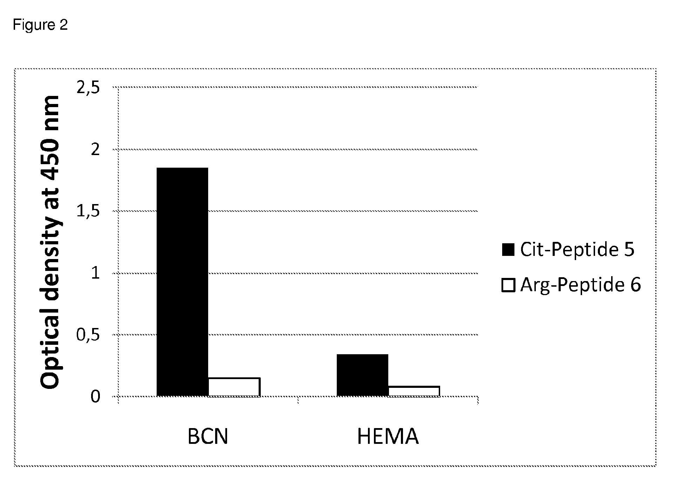

Replacing the clickable monomer in the coating with 2-hydroxyethyl methacrylate resulted in low signals on both citrulline- and arginine-containing peptides (peptides 5 and 6; figure 2). This confirmed that the results seen with the coating containing BCN functionalized monomers were not caused by non-specific absorption to the coating, but were indeed due to specific conjugation between the clickable monomers and the azide-functional peptides.

### Legend to the figures

Figure 1. I)Surface immobilization of a diagnostic peptide using click reaction (I) and subsequent heterogeneous sandwich ELISA (II-IV): II) The primary antibody from the sample of the patient binds to the epitope (triangle) of the immobilized antigen and thereby becomes immobilized itself. III) A secondary enzyme-labeled antibody binds to the primary antibodies which have been bound. IV) The bound enzyme converts substrate 'A' into product 'B' which can be measured by optical density which is proportional to the amount of primary antibody present in the patient's sample.
Figure 2. ELISA Optical Density (OD) measurement at 450 nm. Measurements were carried out for microtiter plates (96 wells) prepared with either BCN methacrylate 2 or 2-hydroxyethyl methacrylate (HEMA). Each microtiter plate was functionalized with either citrulline-containing peptide 5 or arginine-containing peptide 6.

### Examples

*Reagents:* All purchased chemicals were obtained from common commercial sources and used as received. Prop-2-yn-1-yl methacrylate was purchased from *Alfa Aesar.* A monoclonal human anti-citrulline antibody (MQR2.101) was provided by *ModiQuest Research.* Tetrahydrofuran (THF) was distilled from sodium and benzophenone. Dichloromethane (CH₂Cl₂) was distilled from CaH₂ and triethylamine (Et₃N) was dried by distillation from CaCl₂. Water was demineralized prior to use. MilliQ grade water was obtained using the *Labconco Water Pro PS.* The HCl solution in EtOAc was prepared by saturation of EtOAc with chlorine gas. Nunc Lockwell MaxiSorp microtiter plates (96 wells) were used for the coating and subsequent functionalization experiments.

*Instrumentation:* NMR spectra were recorded on a *Varian Inova 400* (400 MHz for ¹H). ¹H-NMR chemical shifts (δ) are reported in parts per million (ppm) relative to a residual proton peak of the solvent: δ = 3.31 for CD₃OD and δ = 7.26 for CDCl₃. Multiplicities are reported as: s (singlet), d (doublet), t (triplet), q (quartet), p (pentet), se (sextet), dd (double doublet), dt (double triplet), tt (triple triplet), ddd (double, double doublet) or m (multiplet). Broad peaks are indicated by br. Coupling constants are reported as a *J*-value in Hertz (Hz). The number of protons (n) for a given resonance is indicated as nH, and is based on spectral integration values. ESI-MS analysis was performed using *Thermo Scientific Advantage LCQ* Linear-Ion trap Electrospray (LRMS). FT-IR spectra were recorded on an *ATI Matson Genesis Series FT-IR* spectrometer fitted with an ATR cell. Vibrations (v) are given in cm⁻¹. As UV source an UVA Spot 400T from *Hönle UV Technology* outfitted with a Quartz filter and a Hönle 400 H/2 lamp was used. Thin layer chromatography (TLC) was carried out on *Merck* precoated silica gel 60 F-254 plates (layer thickness 0.25 mm). Compounds were visualized by UV, KMnO₄, ninhydrin or iodine coloring. Column chromatography was performed using *Acros* silica gel (0.035-0.070 mm, pore diameter ca. 6 nm).

*Synthesis of (1R,8S,9r,Z)-Ethyl bicyclo[6.1.0]non-4-ene-9-carboxylate and (1R,8S,9s,Z)-ethyl bicyclo[6.1.0]non-4-ene-9-carboxylate:* Prepared according to a modified literature procedure.¹³ Quantities used: 1,5-cyclooctadiene (19.6 mL, 160 mmol) and Rh₂(OAc)₄ (380 mg, 0.86 mmol) in CH₂Cl₂ (15 mL); ethyl diazoacetate (2.1 mL, 20 mmol) in CH₂Cl₂ (12 mL). After eluting the excess of 1,5-cyclooctadiene from silica with EtOAc/heptane (1/200), the mixture of the *exo-* and endo-diastereomers was not purified any further, wich afford a colorless oil (2.4 g, 61%) containing the *exo-* and endo-diastereomers in a ratio of 2:1.

*exo*:¹H NMR (CDCl₃, 400 MHz): δ 5.68-5.60 (m, 2H), 4.10 (q, J = 7.2 Hz, 2H), 2.35-2.27 (m, 2H), 2.24-2.16 (m, 2H), 2.13-2.04 (m, 2H), 1.59-1.53 (m, 2H), 1.53-1.43 (m, 2H), 1.25 (t, J= 7.2 Hz, 3H), 1.18 (t, J= 4.8 Hz, 1H).

*endo*: ¹H NMR (CDCl₃, 400 MHz): δ 5.65-5.57 (m, 2H), 4.12 (q, J = 7.2 Hz, 2H), 2.53-2.46 (m, 2H), 2.25-2.16 (m, 2H), 2.10-2.01 (m, 2H), 1.87-1.79 (m, 2H), 1.70 (t, J = 8.8 Hz, 1H), 1.43-1.34 (m, 2H), 1.26 (t, J = 7.2 Hz, 3H).

*Synthesis of (1R,8S,9r)-Bicyclo[6.1.0]non-4-yn-9-ylmethanol (exo-**1**) and (1R,8S,9s)-Bicyclo[6.1.0]non-4-yn-9-ylmethanol (endo-**1**):* Prepared according to a modified literature procedure.¹³ Quantities used: 2:1 mixture of (1 R,8S,9r,Z)-Ethyl bicyclo[6.1.0]non-4-ene-9-carboxylate and (1 R,8S,9s,Z)-ethyl bicyclo[6.1.0]non-4-ene-9-carboxylate (240 mg, 1.24 mmol) in Et₂O (5 mL); LiAlH₄ (56 mg, 1.46 mmol) in Et₂O (5 mL); Br₂ (69 µL, 1.02 mmol) in CH₂Cl₂ (1 mL); KO*t*Bu (4.6 mL, 1 M in THF, 4.60 mmol). This afforded the product as a white solid (130 mg, 79%) containing the *exo-* and *endo-*diastereomers in a ratio of 2:1.

exo-**1**:¹H NMR (CDCl₃, 400 MHz): δ: 3.54 (d, J= 6.4 Hz, 2H), 2.44-2.40 (m, 2H), 2.32-2.25 (m, 2H), 2.18-2.14 (m, 2H), 1.88 (bs, 1H), 1.44-1.34 (m, 2H), 0.74-0.63 (m, 3H).

*endo-***1***:* ¹H-NMR (CDCl₃, 400 MHz): δ: 3.73 (d, J= 8.0 Hz, 2H), 2.35-2.20 (m, 6H), 1.66-1.56 (m, 2H), 1.39- 1.30 (m, 1H), 1.18 (bs, 1H), 0.99-0.90 (m, 2H).

*Synthesis of (1R,8S,9r)-Bicyclo[6.1.0]non-4-yn-9-yl methacrylate (exo-**2**) and (1R,8S,9s)- Bicyclo[6.1.0]non-4-yn-9-yl methacrylate (endo-**2**):* Alcohol **1** (136 mg, 0.91 mmol) was dissolved in dry CH₂Cl₂ (50 mL) and cooled to 0 ºC. Dry triethylamine (Et₃N, 1.85 mL, 18.10 mmol ) and a drop of 4-dimethylaminopyridine (DMAP) were added. A solution of methacryloyl chloride (116 µL, 1.18 mmol) in dry CH₂Cl₂ (5 mL) was added drop wise. The reaction mixture was allowed to warm to room temperature and stirred for 16 hours. The reaction mixture was washed with saturated aqueous NH₄Cl (3 x 50 mL), and dried with Na₂SO₄. The organic layer was concentrated in vacuo and the crude mixture was purified using column chromatography (EtOAc), affording the product as a colorless oil (130 mg, 70%).

1H NMR (CDCl3, 400 MHz) δ: 6.23 (p, J = 1.0 Hz, 2H), 6.11 (m, 3.5H), 5.82 (dq, J = 1.1, 0.5 Hz, 2H), 5.56 (m, 3.5H), 4.74 (m, 3H), 4.24 (d, J = 8.3 Hz, 2H), 4.08 (d, J = 6.6 Hz, 3.5H), 2.43-2.11 (m, 20H), 2.01 (dd, J = 1.0, 0.6 Hz, 6H), 1.95 (dt, J = 1.0, 0.63 Hz, 11H), 1.69-1.51 (m, 12H), 1.46-1.22 (m, 17.5H), 1.02-0.70 (m, 17.5H).

*Synthesis of imidazole-1-sulfonyl azide hydrochloride **(3).*** Prepared according to a literature procedure.²³ Quantities used: NaN₃ (3.26 g, 50 mmol) in MeCN (50 mL); sulfuryl chloride (4.1 mL, 50 mmol); imidazole (6.50 g, 95 mmol); HCl in EtOAc (2 M, 40 mL). This affording the product as white needles (7.05 g, 67%).

It should be noted that diazotransfer reagent **3** is shelf stable, but should not be kept as a concentrated aqueous solution for prolonged time due to risk of decomposition.

In accordance with literature:¹H-NMR (400 MHz, D₂O) δ: 7.44 (dd, 1H), 7.85 (dd, 1H), 9.17 (dd, 1H). LRMS (ESI+) *m*/*z* calcd. for C₃H₃N₅O₂S [M-Cl]⁺ 174.0, found 173.9. FTIR Vₘₐₓ film (cm⁻¹): 2362, 2169, 1436, 1196.

*Synthesis of Fmoc-azido-*ε*-norleucine (**4**).* 9-Fluorenylmethyloxycarbonyl-ε-lysine(tert-butoxycarbonyl) (Fmoc-ε-lysine(Boc), 3.0 g, 6.40 mmol) was dissolved in a solution of HCl in EtOAc (2 M, 150 mL) and stirred at room temperature for 2 hours. The white precipitate that formed was isolated by filtration and dried in vacuo overnight to afford Fmoc-lysine as a white solid (2.13 g, 90%), which was used without further purification.

Fmoc-lysine (2.03 g, 5.51 mmol), CuSO₄ (88 mg, 0.35 mmol) and K₂CO₃ (2.46 g, 17.83 mmol) were dissolved in MeOH (70 mL). Imidazole-1-sulfonyl azide hydrochloride (3, 1.40 g, 6.70 mmol) was added in small portions and the suspension was stirred overnight at room temperature. The solvent was evaporated in vacuo, and the residue was redissolved in CH₂Cl₂ (50 mL). The solution was washed with aqueous HCl (1 M, 50 mL), and the aqueous phase was extracted with CH₂Cl₂ (3 x 50mL). The crude mixture was purified using gradient column chromatography (CH₂Cl₂/MeOH/AcOH from 96.5%/3%/0.5% → 93.5%/6%/0.5%) and lyophilized from dioxane, resulting in a colorless oil (1.94 g, 89%).

In accordance with literature:^{24 1}H-NMR (400 MHz, CDCl₃) δ:7.76 (d, J = 7.5 Hz, 2H), 7.59 (d, J= 6.9 Hz, 2H), 7.40 (t, J= 7.5 Hz, 2H), 7.31 (t, J= 7.5 Hz, 2H), 5.34 (d, J = 7.8 Hz, 1H), 4.54 (br s, 1H), 4.43 (d, J = 6.6 Hz, 2H), 4.22 (t, J = 6.6 Hz, 1H), 3.27 (t, *J*= 6.6 Hz, 2H), 1.3-2.0 (m, 2H). HRMS (ESI+) m/zcalcd for C₂,H₂₃N₄O₈[M+H]⁺ 395.1719, found 395.1718.

*Synthesis of Peptides **5** and **6***: Prior to the peptide synthesis, the Breipohl resin^{25,26} was swollen for 20 minutes in DMF. Stepwise solid-phase peptide synthesis (SPPS) was performed using standard Fmoc-chemistry: Each peptide coupling was executed using 3 equiv. of the appropriate Fmoc-protected amino acid together with diisopropylcarbodiimide (DIPCDI, 3.3 equiv.) and *N*-hydroxybenzotriazole (HOBt, 3.6 equiv.) in DMF for approximately 45 minutes. Fmoc-groups were removed with piperidine in DMF (20% v/v, 3 x 6 min). In the final step, the N-terminus was reacted with 3 equiv. of (+)-Biotin 4-nitrophenyl ester in DMF for 45 minutes.

The peptide was cleaved from the resin by treatment with trifluoroacetic acid/H₂O/triisopropylsilane (92.5/2.5/5.0, v/v) for 4 h. After precipitation in diethyl ether, the peptide was lyophilized from acetic acid. The peptide was characterized using HPLC, FTIR and LRMS (ESI+) (data not shown).

*Immunoreactivity of peptides.* The reactivity of the clickable coatings was subsequently investigated using an azido-functional peptide containing citrulline (peptide 5), a known epitope for specific recognition by a proprietary monoclonal human ACA MQR2.101 available from ModiQuest Research. The corresponding arginine analog (peptide 6) was synthesized as a negative control. To this end, Fmoc-lysine(Boc) was deprotected with HCl in EtOAc and converted into an azide derivative via a diazo transfer reaction using imidazole-1-sulfonyl azide hydrochloride (3) (Scheme 2).[23, 27] The resulting Fmoc-protected azidonorleucine (4) was bound to a Breipohl resin and the consecutive amino acids were built in a stepwise fashion by solid-phase peptide synthesis (SPPS).[25, 26] Introduction of a biotin group on the N-terminus of the peptides containing either arginine or citrulline, using an activated p-nitrophenyl ester, resulted in peptides 5 and 6 respectively (Scheme 2). The peptides were finally cleaved from the resin using a mixture of trifluoroacetic acid, water and triisopropylsilane. Radical scavengers were omitted from the cleavage mixture, because of the possible reduction of azides under the influence of 1,2-ethanedithiol (EDT).[28].

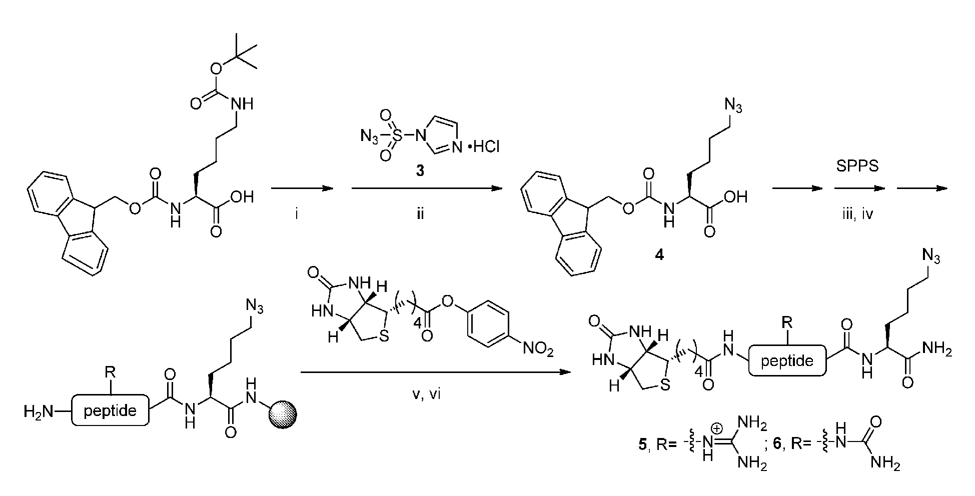

**Scheme 2.** Synthesis of Fmoc-protected azidonorleucine and subsequent incorporation into peptides 5 and 6. *Reagents and conditions:* i) HCl, EtOAc, r.t., 2 h (90%); ii) CuSO₄, K₂CO₃, MeOH, r.t., 16 h (89%); iii) Fmoc-protected amino acid, DIPCDI, HOBt, DMF, r.t., 45 min; iv) piperidine, DMF, r.t., 3 x 6 min; v) DMF, r.t., 45 min; vi) trifluoroacetic acid, H₂O, triisopropylsilane, r.t., 4 h.

As a control, peptides 5 and 6 were provided with a biotin function on their N-terminus and tested for reactivity with the MQR2.101 antibody. Peptide 5 and 6 were immobilized on a avidin-functional microtiter plate and the resulting microtiter plates were evaluated for their potential to bind ACA. After having confirmed that the citrullinated peptide 5 was recognized by human ACA and peptide 6 was not, thepeptides were tested in the click reaction.

*Coating application:* A monomer mixture of poly(ethylene glycol) dimethacrylate (3000 µL), ethylene glycol dimethacrylate (2000 µL), 2-hydroxyethyl methacrylate (3700 µL) and Irgacure 1000 (300 µL) was prepared. This monomer mixture was subsequently mixed with BCN methacrylate 2 in a 9:1 ratio (v/v), affording a stock mixture containing 30% v/v poly(ethylene glycol) dimethacrylate, 20% v/v ethylene glycol dimethacrylate, 37% v/v 2-hydroxyethyl methacrylate, 10% v/v of the clickable monomer (BCN methacrylate) and 3% v/v of initiator. The stock mixtures were diluted in methanol to a final monomer content of 5% (v/v).

Wells of a microtiter plates (96 wells) were filled with 100 µL of coating solution and shaken gently to wet the walls completely. Excess mixture was removed by turning the microtiter plate upside down and forcibly knocking the liquid out, taking care not to cross-contaminate the wells. The liquid film, which remained in the wells, was polymerized using a strong UV source that was positioned 20 centimeters above the sample for 60 seconds. After 30 minutes the UV exposure was repeated in order to improve the clarity and stability of the coating.

*Typical procedure for peptide immobilization on a coating containing **2**:* 0.18 mM solutions of peptides **5** and **6** were prepared by first dissolving the peptides in DMSO and then adding H₂O to a final ratio of 2:1 (v/v). Each of the wells was filled with 80 µL of the peptide solution. The microtiter plate (96 wells) was covered with aluminum foil and left to react at room temperature for 2 hours while gently shaken at 300 rpm. The coupling reaction was stopped by removing the reaction mixtures from the wells and washing the microtiter plate with phosphate buffer (3 x PBS, 10 mM, pH 7.2) followed by phosphate buffered saline Tween-20 solution (3 x PBST, 10 mM, pH 7.2, 0.05% Tween-20) and finally PBS buffer (3 x). The microtiter plate was stored in the fridge while filled with PBS buffer. The extent to which the peptides were coupled to the reactive coating was finally evaluated in an ELISA assay.

*Sandwich ELISA protocol:* The microtiter plate (96 wells) was washed with a phosphate buffered saline Tween-20 solution (3 x PBST, 10 mM, pH 7.2, 0.05% Tween-20; 200 µL/well), then blocked with 1% Bovine Albumin (BSA) in PBST
(380 µL/well) for 1 hour at room temperature. After three additional washes with PBST, 100 µL of human anti-citrulline antibody solution (MQR2.101; 100 ng/100 µL PBST + 1% BSA) was added per well and incubated for 1 hour at room temperature. After three washes with PBST, 100 µl/well of the secondary HRP labeled antibody (monoclonal rabbit anti-Human IgG-HRP diluted 1/2000 in PBST + 1% BSA) was added to the microtiter plate for an additional incubation of 1 hour at room temperature. After three final PBST and four PBS washes, the microtiter plate was developed with 100 µl/well 3,3',5,5'-tetramethylbenzidine (TMB) solution *(Tebu-Bio)* for 5 minutes at room temperature in the dark. The reaction was stopped by adding 100 µl/well 2 M H₂SO₄, and the absorbance was read at 450 nm.

### References

1. Ivanov, V. S.; Suvorova, Z. K.; Tchikin, L. D.; Kozhich, A. T.; Ivanov, V. T., Effective Method for Synthetic Peptide Immobilization That Increases the Sensitivity and Specificity of Elisa Procedures. J. Immunol. Methods 1992, 153, (1-2), 229-233.
2. Blake, C.; Gould, B. J., Use of Enzymes in Immunoassay Techniques - a Review. Analyst 1984, 109, (5), 533-547.
3. Smolen, J. S.; Steiner, G., Are autoantibodies active players or epiphenomena? Current Opinion in Rheumatology 1998, 10, (3), 201-206.
4. van Boekel, M. A. M.; Vossenaar, E. R.; van den Hoogen, F. H. J.; van Venrooij, W. J., Autoantibody systems in rheumatoid arthritis: specificity, sensitivity and diagnostic value. Arthritis Research 2002, 4, (2), 87-93.
5. Vossenaar, E. R.; Zendman, A. J. W.; van Venrooij, W. J.; Pruijn, G. J. M., PAD, a growing family of citrullinating enzymes: genes, features and involvement in disease. Bioessays 2003, 25, (11), 1106-1118.
6. Butler, J. E., Solid supports in enzyme-linked immunosorbent assay and other solid-phase immunoassays. Methods 2000, 22, (1),4-23.
7. Satoh, A.; Kojima, K.; Koyama, T.; Ogawa, H.; Matsumoto, I., Immobilization of saccharides and peptides on 96-well microtiter plates coated with methyl vinyl ether-maleic anhydride copolymer. Analytical Biochemistry 1998, 260, (1), 96-102.
8. Rostovtsev, V. V.; Green, L. G.; Fokin, V. V.; Sharpless, K. B., A stepwise Huisgen cycloaddition process: Copper(I)-catalyzed regioselective "ligation" of azides and terminal alkynes. Angewandte Chemie-International Edition 2002, 41, (14),2596-+.
9. Debets, M. F.; van Berkel, S. S.; Schoffelen, S.; Rutjes, F. P. J. T.; van Hest, J. C. M.; van Delft, F. L., Aza-dibenzocyclooctynes for fast and efficient enzyme PEGylation via copper-free (3+2) cycloaddition. Chemical Communications 2010, 46, (1), 97-99.
10. Baskin, J. M.; Prescher, J. A.; Laughlin, S. T.; Agard, N. J.; Chang, P. V.; Miller, I. A.; Lo, A.; Codelli, J. A.; Bertozzit, C. R., Copper-free click chemistry for dynamic in vivo imaging. Proceedings of the National Academy of Sciences of the United States of America 2007, 104, 16793-16797.
11. van Berkel, S. S.; Dirks, A. J.; Debets, M. F.; van Delft, F. L.; Cornelissen, J. J. L. M.; Nolte, R. J. M.; Rutjes, F. P. J. T., Metal-free triazole formation as a tool for bioconjugation. Chembiochem 2007, 8, (13), 1504-1508.
12. Ning, X. H.; Guo, J.; Wolfert, M. A.; Boons, G. J., Visualizing metabolically labeled glycoconjugates of living cells by copper-free and fast huisgen cycloadditions. Angewandte Chemie-International Edition 2008, 47, (12), 2253-2255.
13. Dommerholt, J.; Schmidt, S.; Temming, R.; Hendriks, L. J. A.; Rutjes, F. P. J. T.; van Hest, J. C. M.; Lefeber, D.; Friedl, P.; van Delft, F. L., Angewandte Chemie-International Edition 2010, in press.
14. Canalle, L. A.; van Berkel, S. S.; de Haan, L. T.; van Hest, J. C. M., Copper-Free Clickable Coatings. Advanced Functional Materials 2009, 19, (21), 3464-3470.
15. Daugaard, A. E.; Hvilsted, S.; Hansen, T. S.; Larsen, N. B., Conductive polymer functionalization by click chemistry. Macromolecules 2008, 41, (12), 4321-4327.
16. Fleischmann, S.; Hinrichs, K.; Oertel, U.; Reichelt, S.; Eichhorn, K. J.; Voit, B., Modification of polymer surfaces by click chemistry. Macromolecular Rapid Communications 2008, 29, (12-13), 1177-1185.
17. He, X. Z.; Ma, J. Y.; Jabbari, E., Effect of Grafting RGD and BMP-2 Protein-Derived Peptides to a Hydrogel Substrate on Osteogenic Differentiation of Marrow Stromal Cells. Langmuir 2008, 24, (21), 12508-12516.
18. Im, S. G.; Bong, K. W.; Kim, B. S.; Baxamusa, S. H.; Hammond, P. T.; Doyle, P. S.; Gleason, K. K., Patterning Nanodomains with Orthogonal Functionalities: Solventless Synthesis of Self-Sorting Surfaces. Journal of the American Chemical Society 2008, 130, (44), 14424-14425.
19. Li, Y.; Zhang, W. X.; Chang, J.; Chen, J. C.; Li, G. T.; Ju, Y., "Click" on conducting polymer coated electrodes: A versatile platform for the modification of electrode surfaces. Macromolecular Chemistry and Physics 2008, 209, (3), 322-329.
20. Nandivada, H.; Chen, H. Y.; Bondarenko, L.; Lahann, J., Reactive polymer coatings that "click"". Angewandte Chemie-International Edition 2006, 45, (20), 3360-3363.
21. Rengifo, H. R.; Chen, L.; Grigoras, C.; Ju, J. Y.; Koberstein, J. T., "Click-Functional" block copolymers provide precise surface functionality via spin coating. Langmuir 2008, 24, (14), 7450-7456.
22. Engvall, E.; Perlmann, P., Enzyme-Linked Immunosorbent Assay, Elisa .3. Quantitation of Specific Antibodies by Enzyme-Labeled Anti-Immunoglobulin in Antigen-Coated Tubes. Journal of Immunology 1972, 109, (1), 129-135.
23. Goddard-Borger, E. D.; Stick, R. V., An efficient, inexpensive, and shelf-stable diazotransfer reagent: Imidazole-1-sulfonyl azide hydrochloride. Organic Letters 2007, 9, 3797-3800.
24. Agnew, H. D.; Rohde, R. D.; Millward, S. W.; Nag, A.; Yeo, W. S.; Hein, J. E.; Pitram, S. M.; Tariq, A. A.; Burns, V. M.; Krom, R. J.; Fokin, V. V.; Sharpless, K. B.; Heath, J. R., Iterative In Situ Click Chemistry Creates Antibody-like Protein-Capture Agents. Angewandte Chemie-International Edition 2009, 48, (27), 4944-4948.
25. Breipohl, G.; Knolle, J.; Stüber, W., Synthesis and application of acid labile anchor groups for the synthesis of peptide amides by Fmoc-Solid-Phase Peptide synthesis. Int J. Pept. Protein Res. 1989, 34, (4), 262-267.
26. Stüber, W.; Knolle, J.; Breipohl, G., Synthesis of peptide amides by Fmoc-Solid-Phase Peptide Synthesis and acid labile anchor groups. Int. J. Pept. Protein Res. 1989, 34, (3), 215-221.
27. van Dongen, S. F. M.; Teeuwen, R. L. M.; Nallani, M.; van Berkel, S. S.; Cornelissen, J. J. L. M.; Nolte, R. J. M.; van Hest, J. C. M., Single-Step Azide Introduction in Proteins via an Aqueous Diazo Transfer. Bioconjugate Chemistry 2009, 20, (1), 20-23.
28. Schneggenburger, P. E.; Worbs, B.; Diederichsen, U., Azide reduction during peptide cleavage from solid support - The choice of thioscavenger? Journal of Peptide Science 2009, 16, (1), 10-14.
29. Kroneck, P. M. H.; Vortisch, V.; Hemmerich, P., Model studies on the coordination of copper in biological systems - the deprotonated peptide nitrogen as a potential binding site for copper(II). European Journal of Biochemistry 1980, 109, (2), 603-612.

## Claims

1. Method for immobilizing a bicycloalkyne to a solid support wherein a UV-reactive monomer is attached to the bicycloalkyne to obtain a UV-reactive clickable monomer, where after the UV-reactive clickable monomer is covalently linked to the solid support by means of UV irradiation in the presence of a photoinitiator.

2. Method according to claim 1 wherein the bicycloalkyne is a bicyclononyne.

3. Method according to claim 2 wherein the bicyclononyne is bicyclo[6.1.0]non-4-yne.

4. Method according to any of claims 1 ― 3 wherein the UV-reactive monomer is selected from the group consisting of acrylates, methacrylates and acrylamides.

5. Method according to any of claims 1 ― 4 wherein the solid support comprises a material selected from the group consisting of glass, paper, metal and polymers.

6. Method according to claim 5 wherein the polymer is selected from the group consisting of polystyrene and polymethylmethacrylate.

7. Bicycloalkyne, covalently attached to a solid support.

8. Functionalized solid support obtainable by a method according to claims 1 ― 6.

9. Enzyme-linked immunosorbent assay wherein an analyte is covalently attached to a solid support through an azide-alkyne 1,3 dipolar cycloaddition.


SEQUENCE LISTING

<110>  MODIQUEST B.V.

<120>  METHOD FOR PREPARING A REACTIVE COATING

<130>  209 EPP0

<160>  2

<170>  PatentIn version 3.5

<210>  1
<211>  21
<212>  PRT
<213>  homo sapiens
<220>
<221>  MISC_FEATURE
<222>  (8)..(8)
<223>  X = Citrulline
<220>
<221>  MISC_FEATURE
<222>  (15)..(15)
<223>  X = Citrulline

<400>  1
Leu Ser Glu Gly Gly Gly Val Xaa Gly Pro Arg Val Val Glu Xaa His
1               5                   10                  15

Gln Ser Gln Cys Lys
            20

<210>  2
<211>  21
<212>  PRT
<213>  homo sapiens

<400>  2

Leu Ser Glu Gly Gly Gly Val Arg Gly Pro Arg Val Val Glu Arg His

1                    5                        10                        15

Gln Ser Gln Cys Lys
                20

SEQUENCE LISTING

<110>  MODIQUEST B.V.

<120>  METHOD FOR PREPARING A REACTIVE COATING

<130>  209 EPPO

<160>  2

<170>  PatentIn version 3.5

<210>  1
<211>  21
<212>  PRT
<213>  homo sapiens


<220>
<221>  MISC_FEATURE
<222>  (8)..(8)
<223>  X = Citrulline

<220>
<221>  MISC_FEATURE
<222>  (15)..(15)
<223>  X = Citrulline

<400>  1

Leu Ser Glu Gly Gly Gly Val Xaa Gly Pro Arg Val Val Glu Xaa His
1               5                   10                  15


Gln Ser Gln Cys Lys
            20


<210>  2
<211>  21
<212>  PRT
<213>  homo sapiens

<400>  2

Leu Ser Glu Gly Gly Gly Val Arg Gly Pro Arg Val Val Glu Arg His
1               5                   10                  15


Gln Ser Gln Cys Lys
            20